# EUROPÄISCHE PATENTSCHRIFT

(11) **EP 0 845 251 B1**
(45) Veröffentlichungstag und Bekanntmachung des Hinweises auf die Patenterteilung: **26.09.2001**
(21) Anmeldenummer: 98104084.3
(22) Anmeldetag: 21.10.1993
(51) Int. Cl.: A61F 2/36

(54) **Bausatz für eine modulare Femurkapfprothese, insbesondere eine Reoperationsprothese, und Femurkopfprothese aus einem derartigen Bausatz**
Construction kit for a modular femoral head prosthesis, especially a reoperation prosthesis, and femoral head prosthesis made from such a kit
Eléments de construction pour une prothèse de tête fémorale modulaire, en particulier une prothèse de réopération, et prothèse de tête fémorale obtenue avec detels éléments

(43) Veröffentlichungstag der Anmeldung: 03.06.1998
(62) Teilanmeldung aus: 93810738.0
(73) Patentinhaber: Sulzer Orthopädie AG, 6340 Baar (CH)
(72) Erfinder: Baur, Nikolaus, 8247 Flurlingen (CH); Lamprecht, Stefan, 8309 Birchwil (CH)
(74) Vertreter: Sulzer Management AG

(56) Entgegenhaltungen:
- EP-A- 0 019 042
- EP-A- 0 304 756
- EP-A- 0 399 920
- DE-A- 2 933 230
- DE-A- 3 033 227
- DE-A- 3 406 358
- DE-A- 3 736 304
- DE-C- 3 805 303
- FR-A- 2 605 514
- FR-A- 2 622 791
- FR-A- 2 633 509
- US-A- 4 051 559

## Beschreibung

Die Erfindung betrifft einen Bausatz für eine modulare Femurkopfprothese, insbesondere eine Reoperationsprothese, enthaltend einen in einem Femurknochen verankerbaren distalen Schaftteil und einen auf dessen proximaler Endpartie mittels einer Schraubverbindung befestigbaren, mit einer künstlichen Gelenkkugel bestückbaren Halsteil. Ferner betrifft die Erfindung eine aus einem derartigen Bausatz zusammengesetzte Femurkopfprothese.

Derartige Prothesen sind bekannt aus der FR-A-2,605,514 und aus der US-A-4,051,559, sie sind aber auch aus der EP-A-0,399,920 bekannt. Die aus der EP-A-0,339,920 bekannte Femurkopfprothese enthält einen mit einem Aussengewinde versehenen Schaftteil mit einem kegelstumpfförmigen proximalen Fortsatz und einen auf diesen aufsetzbaren Halsteil mit einem gegenüber der Längsachse des Schaftteils abgewinkelten Aufsteckdorn für die Gelenkkugel. Der Halsteil ist durch eine in eine axiale Bohrung des Schaftteils oder eines damit verbundenen Einsatzteils einschraubbare Sicherungsschraube gegen den Fortsatz des Schaftteils verspannbar gehalten. Der Schaftteil der bekannten Endoprothese ist in einem Längenabschnitt seiner axialen Bohrung mit zungenartig ausstellbaren Segmenten ausgeführt, welche über einen in axialer Richtung in die Bohrung einführbaren Verstellteil mit keilförmigen Abstützflächen je gegenüber zwei benachbarten feststehenden Segmenten des Schaftteils radial nach aussen gegen die den Schaftteil umgebende Partie des Femurknochens verspannbar sind. Die auf den Femurknochen zu übertragenden Hauptbelastungskräfte werden jeweils über die gegenüber der Längsachse des Schaftteils lateral versetzte Gelenkkugel in die Femurschaftprothese eingeleitet. Entsprechend werden Femurschaftprothesen der genannten Art jeweils durch relativ grosse dynamische Kräfte und Biegemomente beansprucht. Derartige dynamische Beanspruchungen können bei bisherigen Endoprothesen der genannten Art zu einer Lockerung der Schraubverbindung zwischen Halsteil und Schaftteil und/oder einer Lockerung der Verankerung des Schaftteils im Femurknochen und damit, z.B. im Bereich einer dübelartigen Aufspreizung des Schaftteils, zu hohen örtlichen Beanspruchungen des Knochengewebes führen.

Die aus der US-A-4,051,559 bekannte Femurkopfprothese umfasst einen im Femurknochen verankerbaren Schaftteil und einen auf dessen proximaler Endpartie mittels einer Schraubverbindung befestigbaren Halsteil, der mit einer an dem Halsteil angeformten Gelenkkugel versehen ist. Die proximale Endpartie des Schaftteils und die auf diese proximale Endpartie aufsetzbare Anschlusspartie des Halsteils sind mit gegeneinander abstützbaren Abstützflächen versehen, die quer zur Längsachse des Schaftteils gestellt sind. Die Anschlusspartie des Halsteils sowie die proximale Endpartie des Schaftteils sind ferner mit sechseckigen Führungsflächen versehen, die in Richtung der Längsachse des Schaftteils orientiert sind und die formschlüssig zusammenführbar sind.

Der Erfindung liegt die Aufgabe zugrunde, einen Bausatz für eine Femurkopfprothese der genannten Art in einer einfachen, robusten Bauweise zu schaffen, welcher aus wenigen, möglichst günstig beanspruchten Bauteilen besteht, insbesondere im Hinblick auf die Biegemomente, welche auf die Prothese wirken.

Diese Aufgabe wird erfindungsgemäss durch einen Bausatz gemäss Anspruch 1 und durch eine Femurkopfprothese gemäss Anspruch 7 gelöst.

Der erfindungsgemäss ausgebildete Bausatz gestattet eine funktionelle Trennung der formschlüssigen, winkelgerechten Positionierung des Halsteils auf dem Schaftteil einerseits und der kraftschlüssigen Verbindung zwischen dem Halsteil und dem in den Femurknochen eingesetzten Schaftteil andererseits. Entsprechend sind die proximale Endpartie des Schaftteils und deren Fortsatz sowie die auf diese Endpartie aufsetzbare Anschlusspartie des Halsteils und deren Aussparung jeweils mit einem zumindest annähernd rechteckigen Querschnitt ausgebildet, dessen grössere Längenabmessung im wesentlichen in lateraler Richtung des Femurknochens verläuft. Dadurch sind die für die Aufnahme des grössten Biegemoments massgebenden Flächen optimiert, wobei gleichzeitig die aufgrund von Druckkräften auftretende Flächenpressung innerhalb vorbestimmter Grenzen gehalten werden kan.

Ein weiterer Vorteil der erfindungsgemässen Ausführung besteht darin, dass durch die über die Dehnschaftschraube gegeneinander verspannbaren, mit hoher Flächenpressung aneinanderliegenden Abstützflächen des Halsteils und des Schaftteils ein Austreten von zwischen diesen Teilen entstehendem Abrieb verhindert wird. Der erfindungsgemässe Bausatz kann aus relativ wenigen Bauteilen bestehen, welche einem Vorrat von mehreren, mit unterschiedlichen Querschnitten und/oder unterschiedlichen, z.B. zentimeterweise abgestuften Längenabmessungen ausgeführten Schaftteilen und von mehreren, z.B. je in zwei, mit unterschiedlichen Abmessungen ausgeführten Halsteilen und Dehnschaftschrauben entnommen und zu einer den gegebenen anatomischen Verhältnissen entsprechenden Femurkopfprothese zusammengesetzt werden können.

In den abhängigen Patentansprüchen sind vorteilhafte Ausgestaltungen des Erfindungsgegenstandes angegeben.

Weitere Merkmale und Einzelheiten ergeben sich aus der folgenden Beschreibung in Form in der Zeichnung schematisch dargestellten Ausführungsbeispielen nach der Erfindung, in Verbindung mit-den Patentansprüchen. In der Zeichnung zeigen:
- Fig. 1: Eine erfindungsgemäss ausgebildete Femurkopf-Endoprothese in einer Teilansicht mit Teilschnitt;
- Fig. 2: die Endoprothese in einem Längsschnitt entsprechend der Linie II-II in Fig. 1;
- Fig. 3: die Endoprothese in einer Draufsicht;
- Fig. 4: die Endoprothese in einem Querschnitt entsprechend der Linie IV-IV in Fig. 1;
- Fig. 5: einen Querschnitt entsprechend der Linie V-V in Fig. 1;
- Fig. 6: eine Endoprothese in einer abgewandelten Ausführungsform, in einer der Fig. 2 entsprechenden Darstellung.

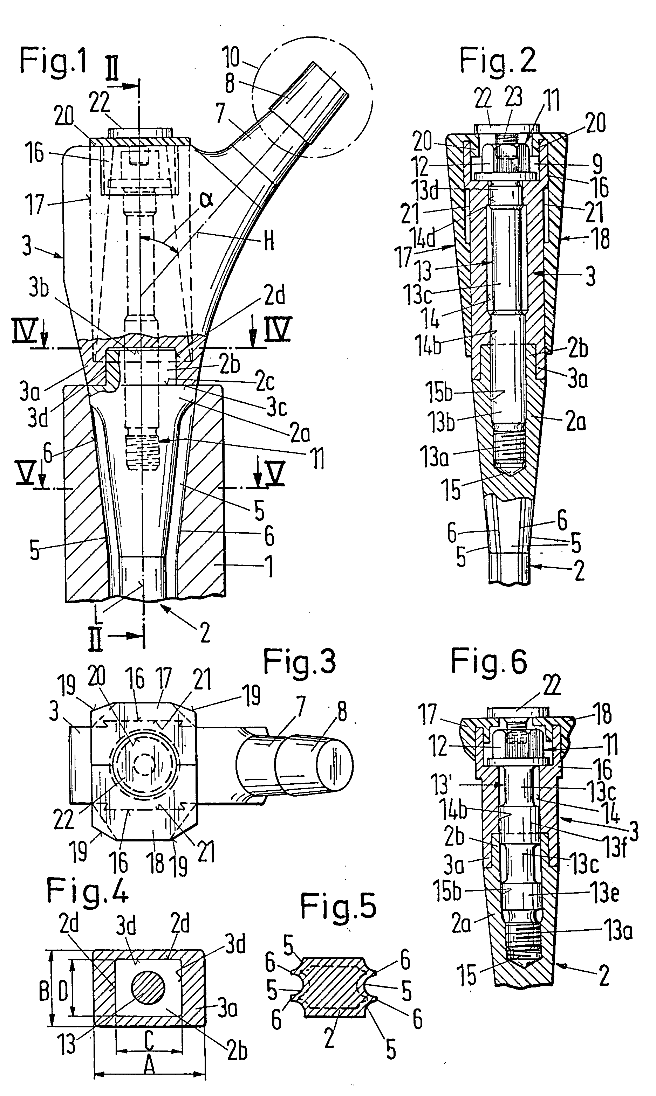

Die Femurkopfprothese nach den Figuren 1 bis 5 ist Teil einer Reoperationsprothese, die als Zweit- oder Drittprothese zur Verwendung bei Patienten vorgesehen ist, bei denen eine entsprechende, früher eingesetzte Endoprothese aus irgendwelchen Gründen, z.B. wegen Lockerung ihrer Verankerung im Knochengewebe, reoperiert und ersetzt werden muss. Die dargestellte Femurkopfprothese enthält einen in einen Femurknochen 1 implantierbaren und in diesem mittels Knochenzement oder, wie dargestellt, zementfrei verankerbaren distalen Schaftteil 2 und einen auf dessen proximale Endpartie 2a aufsetzbaren Halsteil 3 aus einem körperverträglichen Metall, z.B. Titan. Der Halsteil 3 ist als Ersatz für eine fehlende Knochenpartie, beim dargestellten Beispiel die proximale Endpartie des Femurknochens 1 vorgesehen, welche, insbesondere in Reoperationsfällen, etwa infolge von Abbauerscheinungen und Demineralisation des Knochengewebes häufig teilweise oder, wie dargestellt, vollständig reseziert werden muss. Der Schaftteil 2 enthält einen in Richtung seiner Längsachse L in den Femurknochen 1 einsetzbaren, von seiner Endpartie 2a gegen seine nicht dargestellte distale Endpartie konisch sich verjüngenden Schaftkörper und einen an der Endpartie ausgebildeten zapfenartigen Fortsatz 2b, der in eine entsprechende, am Halsteil 3 ausgebildete Aussparung 4 vorsteht. Der Schaftkörper 2 ist mit mehreren, zumindest über einen Teil seiner Längserstreckung verlaufenden Längsnuten 5 bzw. zwischen diesen ausgebildeten, in das Knochengewebe eindringenden schneidenartigen Längsrippen 6 ausgeführt und durch diese im Femurknochen 1 gegen Verdrehen gesichert gehalten.

Der Halsteil 3 enthält einen dem resezierten Ende des Femurknochens 1 entsprechenden Hauptkörper mit einer auf die Endpartie 2a des Schaftteils 2 aufsetzbaren Anschlusspartie 3a und einem seitlich angeformten, bezüglich der Längsachse L des Schaftteils 2 lateral abstehend positionierbaren Aufsteckdorn 7, dessen Längsachse H unter einem Winkel α zur Längsachse L geneigt ist. Der Aufsteckdorn 7 endet in einem Konus 8, auf den in bekannter Weise eine gegenüber der Längsachse lateral versetzte, strichpunktiert dargestellte künstliche Gelenkkugel 10 aufsetzbar ist. Die Endpartie 2a des Schaftteils 2 und zumindest die Anschlusspartie 3a, darstellungsgemäss der ganze Hauptkörper, des Halsteils 3 sind je mit einem zumindest annähernd rechteckförmigen Querschnitt und mit entsprechenden, quer zur Längsachse L gestellten, gegeneinander abstützbaren rechteckförmigen Abstützflächen 2c bzw. 3c ausgeführt, welche je mit in lateraler Richtung orientierter grosser Längenabmessung A und in transversaler Richtung orientierter kleinerer Breitenabmessung B angeordnet sind. Der Fortsatz 2b des Schaftteils 2 und die Aussparung 3b des Schaftteils 3 sind ebenfalls je mit einem entsprechenden, annähernd rechteckförmigen Querschnitt und mit in Richtung der Längsachse L formschlüssig zusammenführbaren Mantelflächen 2d bzw. 3d ausgeführt, durch welche der Schaftteil 2 und der Halsteil 3 in einer bezüglich der Lage der Gelenkkugel 10 definierten Stellung miteinander kuppelbar sind.

Der Halsteil 3 ist auf dem Schaftteil 2 mittels einer Dehnschaftschraube 11 befestigt, welche einen in einer Vertiefung 9 des Halsteils 3 versenkt abstützbaren Kopf 12 und einen Schraubenschaft 13 aufweist, der eine Bohrung 14 des Halsteils 3 und eine axiale Bohrung 15 des Schaftteils 2 durchsetzt. Der Schraubenschaft 13 ist mit einem in eine Gewindebohrung des Schaftteils 2 einschraubbaren Gewindeabschnitt 13a und zwei gegenüber diesem verdickten, im Abstand voneinander angeordneten Führungsabschnitten 13b und 13d sowie mit einem diese verbindenden, elastisch vorspannbaren Dehnabschnitt 13c ausgeführt, der in bekannter Weise einen Querschnitt aufweist, der kleiner ist als der Kernquerschnitt des Gewindeabschnitts 13a. An den Führungsabschnitten 13b und 13d sind zylindrische Passflächen ausgebildet, welche zum Zusammenwirken mit entsprechenden, an den Bohrungen 14 und 15 ausgebildeten Passflächen bestimmt sind. Gemäss Fig. 2 ist der Führungsabschnitt 13d in der Nähe des Kopfes 13 ausgebildet und wirkt mit einem Bohrungsabschnitt 14d des Halsteils 3 zusammen, während der Führungsabschnitt 13b sich über die Trennstelle zwischen dem Halsteil 3 und dem Schaftteil 2 erstreckt und mit einem Bohrungsabschnitt 14b des Halsteils 3 und einem Bohrungsabschnitt 15b des Schaftteils 2 zusammenwirkt.

Der Halsteil 3 und der Schaftteil 2 sind durch Anziehen der Dehnschaftschraube 11 gegeneinander mit einer Vorspannkraft verspannbar, welche entsprechend einem vorbestimmten grössten Biegemoment aus der am Halsteil 3 gegenüber der Längsachse L lateral versetzt angreifenden, über die Gelenkkugel 10 eingeleiteten Hauptbelastungskraft gewählt werden kann. Die jeweilige Vorspannkraft ist durch das Anzugsdrehmoment der Dehnschaftschraube 11 bestimmt, welches in bekannter Weise an einem Drehmomentenschlüssel auf einen vorbestimmten Höchstwert begrenzt werden kann. Die der Vorspannkraft entsprechende Stützkraft wird ausschliesslich durch die den Fortsatz 2b und die Aussparung 3b umgebenden rechteckförmigem Abstützflächen 2c bzw. 3c übertragen, welche dementsprechend für die Aufnahme der aus der Hauptbelastung resultierenden dynamischen Kräfte und Biegemomente dimensioniert sind.

Beim Aufsetzen des Halsteils 3 auf den Schaftteil 2 werden diese Teile durch die Führungsflächen 2d und 3d des Fortsatzes 2b bzw. der Aussparung 3b zentriert und gegen Verdrehen gesichert. Durch die Dehnschaftschraube 11 wird eine zusätzliche Feinzentrierung des Halsteils 3 und des Schaftteils 2 über die zusammenwirkenden Passflächen der Führungsabschnitte 13b und 13d und der Bohrungsabschnitte 14b und 15b bzw. 14d erzielt, welche zugleich eine von Biegebeanspruchungen freie Führung des Dehnabschnitts 13c der Dehnschaftschraube 11 gewährleisten. Durch die beschriebene Anordnung, welche eine funktionelle Trennung der die Längskräfte übertragenden Teile und der die Querkräfte übertragenden Teile ermöglicht, wird eine formstabile, biegesteife Verbindung zwischen dem in sich biegesteifen Halsteil 3 und dem biegesteifen Endabschnitt des für die Einleitung der zu übertragenden Kräfte in lateraler Richtung verbreiterten des Schaftteils 2 erzielt. Durch diese Ausführung wird auch bei hohen wechselnden Beanspruchungen eine sichere Uebertragung der zwischen der Gelenkkugel 10 und dem Schaftteil 2 wirkenden dynamischen Kräfte und Biegemomente gewährleistet.

Die innerhalb der Aussparung 3b formschlüssig zusammenführbaren Führungsflächen 2d und 3d sind durch die sie umgebenden, unter Vorspannung zusammengepressten Abstützflächen 2c und 3c nach aussen abgedichtet, so dass ein Austreten von gegebenenfalls entstehendem Abrieb in das umgebende Gewebe sicher verhindert werden kann. Durch die für die Aufnahme des grössten Biegemomentes optimierbaren, in Form rechteckiger Hohlquerschnitte ausgeführten Abstützflächen 2c und 3c des Schaftteils 2 und des Halsteils 3 - mit den für das Widerstandsmoment des Querschnitts massgebenden Flächenteilen in den lateralen Randpartien - kann zugleich die bei der Uebertragung der Druckkräfte auftretende Beanspruchung auf Flächenpressung innerhalb vorbestimmter Grenzen gehalten werden. Um eine für unterschiedliche anatomische Verhältnisse geeignete, möglichst universell einsetzbare Ausführung zu erhalten, können die Abstützflächen 2c und 3c je z.B. eine Längenabmessung A = ca. 20 mm bis 30 mm und eine Breitenabmessung B = ca. 15 mm bis 20 mm aufweisen. Der Fortsatz 2b und die Aussparung 3b können entsprechend je z.B. eine Längenabmessung C = ca. 10 bis 20 mm und eine Breitenabmessung D = ca. 10 bis 15 mm aufweisen. Es versteht sich, dass auch andere, den jeweiligen anatomischen Verhältnissen angepasste Abmessungen der Endpartie 2a und der Anschlusspartie 3a möglich sind.

Als Ersatz für eine fehlende, resezierte Knochenpartie können an den Seitenwänden des Halsteils 3, darstellungsgemäss anterior und posterior, backenartige Füllkörper 17 bzw. 18 angebracht werden. Diese können darstellungsgemäss mit abgeschrägten Eckpartien 19 und unterschiedlichen, gegen den Halsteil 2 hin abnehmenden Dicken ausgeführt sowie mit nicht dargestellten Mitteln zum daran Befestigen von Muskeln und Bändern versehen sein. Die Füllkörper 17 und 18, die aus Metall oder, wie dargestellt, aus einem körperverträglichen Kunststoff bestehen können, sind je mit einer auf die proximale Endpartie des Halsteils 3 aufsetzbaren und in deren Vertiefung 9 einführbaren, halbkreisförmigen Kragenpartie 20 und einer nutenartigen Führungsbahn 21 ausgeführt, mit der der betreffende Füllkörper 17 bzw. 18 auf eine an der Seitenwand des Halsteils 3 ausgebildete Führungspartie in Form eines schienenartigen Nockens 16 aufgesetzt und in Richtung der Längsachse L auf die betreffende Seitenwand aufgeschoben werden kann, bis die Kragenpartie 20 in die Vertiefung 9 einrastet. Der Nocken 16 und die Führungsbahn 21 können je mit einem rechteckigen oder, wie dargestellt, einem schwalbenschwanzförmigen Querschnitt ausgeführt sein. Nach einer anderen Ausführungsform können die Führungsbahnen 21 am Halsteil 3 und entsprechende Führungsteile an den Füllkörpern 17, 18 ausgebildet sein.

Zur axialen Sicherung der durch die Kragenpartie 20 zentrierten und durch die Nocken 16 seitlich geführten Füllkörper 17 und 18 kann ein die Vertiefung 9 überdeckender Deckel 22 vorgesehen sein, welcher mit einem in den Schraubenkopf 12 einschraubbaren Gewindezapfen 23 versehen und gegen die Kragenpartie 20 verspannbar ist. Der Deckel 22 verhindert zugleich ein Einwachsen von Gewebe in die Vertiefung 9. Die beschriebene Ausführung der Füllkörper 17 und 18 und des Deckels 23 gestattet dem Operateur die Auswahl der den jeweiligen anatomischen Verhältnissen entsprechenden Füllkörper 17, 18 aus einem Vorrat von in unterschiedlichen Ausführungen bereitgestellten Füllkörpern und die Anbringung der Füllkörper 17, 18 auf dem bereits implantierten Halsteil 3 während einer vorteilhaft späten Operationsphase.

Es ist auch eine Ausführung möglich, bei der nur einer der Füllkörper 17 bzw. 18 auf dem Halsteil 3 angebracht wird. Ferner kann ein entsprechend geformter Füllkörper auch an der lateralen Seitenwand des Halsteils 3 angebracht werden.

Die Ausführung nach Fig. 6 entspricht im wesentlichen der vorstehend beschriebenen Ausführung, wobei der Halsteil 3 eine geringere Bauhöhe und die Dehnschaftschraube 11 einen entsprechend kürzeren Schraubenschaft 13' als beim Beispiel nach den Figuren 1 und 2 aufweist. Der Schraubenschaft 13' ist mit zwei elastisch vorspannbaren Dehnabschnitten 13c und zwei Führungsabschnitten 13e und 13f ausgeführt, wobei der den Gewindeabschnitt 13a anschliessende Führungsabschnitt 13e ausschliesslich mit dem Bohrungsabschnitt 15b des Schaftteils 2 zusammenwirkt und der zwischen den beiden Dehnabschnitten 13c angeordnete Führungsabschnitt 13f sich über die Trennstelle zwischen dem Halsteil 3 und dem Schaftteil 2 erstreckt und mit dem oberen Ende des Bohrungsabschnitts 15b des Schaftteils 2 und dem unteren Ende des Bohrungsabschnitts 14b des Halsteils 3 zusammenwirkt. Entsprechend werden auch bei dieser Ausführung eine Feinzentrierung des Schaftteils 2 und des Halsteils 3 durch den Führungsabschnitt 13f und eine im wesentlichen biegungsfreie Beanspruchung der Dehnschaftschraube 11 erzielt.

Zusammenfassend lässt sich die Erfindung wie folgt beschreiben:
Der Bausatz für eine modulare Femurkopfprothese, insbesondere für eine Reoperationsprothese, enthält einen in einem Femurknochen (1) verankerbaren distalen Schaftteil (2) und einen auf dessen proximaler Endpartie (2a) mittels einer Schraubverbindung befestigbaren, mit einer künstlichen Gelenkkugel (10) bestückbaren Halsteil (3). Die proximale Endpartie (2a) des Schaftteils (2) und eine auf diese proximale Endpartie (2a) aufsetzbare Anschlusspartie (3a) des Halsteils (3) sind mit quer zur Längsachse (L) des Schaftteils (2) gestellten, gegeneinander abstützbaren Abstützflächen (2c,3c) sowie mit in Richtung der Längsachse (L) orientierten, formschlüssig zusammenführbaren Führungsflächen (2d,3d) ausgeführt. Diese Führungsflächen (2d,3d) sind an einem zapfenartigen Fortsatz (2b) an der proximalen Endpartie (2a) des Schaftteils (2) und in einer zur Aufnahme dieses Fortsatzes (2a) ausgebildeten Aussparung (3b) in der Anschlusspartie (3a) des Halsteils (3) ausgebildet. Die Schraubverbindung zum Verbinden von Schaftteil (2) und Halsteil (3) enthält eine mit einer vorbestimmten Vorspannkraft elastisch vorspannbare Dehnschaftschraube (11). Die proximale Endpartie (2a) des Schaftteils (2) und deren Fortsatz (2b) sowie die auf diese Endpartie (2a) des Schaftteils (2) aufsetzbare Anschlusspartie (3a) des Halsteils (3) und deren Aussparung (3b) sind jeweils mit einem zumindest annähernd rechteckförmigen Querschnitt ausgeführt, dessen grössere Längenabmessung (A) im wesentlichen in lateraler Richtung des Femurknochens (1) verläuft.

## Patentansprüche

1. Bausatz für eine modulare Femurkopfprothese, insbesondere für eine Reoperationsprothese, enthaltend einen in einem Femurknochen (1) verankerbaren distalen Schaftteil (2) und einen auf dessen proximaler Endpartie (2a) mittels einer Schraubverbindung befestigbaren, mit einer künstlichen Gelenkkugel (10) bestückbaren Halsteil (3), wobei die Schraubverbindung zum Verbinden von Schaftteil (2) und Halsteil (3) eine mit einer vorbestimmten Vorspannkraft elastisch vorspannbare Dehnschaftschraube (11) enthält, und wobei die proximale Endpartie (2a) des Schaftteils (2) und eine auf diese proximale Endpartie (2a) aufsetzbare Anschlusspartie (3a) des Halsteils (3) mit quer zur Längsachse (L) des Schaftteils (2) gestellten, gegeneinander abstützbaren Abstützflächen (2c,3c) sowie mit in Richtung der Längsachse (L) orientierten, formschlüssig zusammenführbaren Führungsflächen (2d,3d) ausgeführt sind, **dadurch gekennzeichnet, dass** die Führungsflächen (2d,3d) an einem zapfenartigen Fortsatz (2b) an der proximalen Endpartie (2a) des Schaftteils (2) und in einer zur Aufnahme dieses Fortsatzes (2a) ausgebildeten Aussparung (3b) in der Anschlusspartie (3a) des Halsteils (3) ausgebildet sind, und dass die proximale Endpartie (2a) des Schaftteils (2) und deren Fortsatz (2b) sowie die auf diese Endpartie (2a) des Schaftteils (2) aufsetzbare Anschlusspartie (3a) des Halsteils (3) und deren Aussparung (3b) jeweils mit einem zumindest annähernd rechteckförmigen Querschnitt ausgeführt sind, dessen grössere Längenabmessung (A) im wesentlichen in lateraler Richtung des Femurknochens (1) verläuft.

2. Bausatz nach Anspruch 1, **dadurch gekennzeichnet, dass** die Dehnschaftschraube (11) mit zwei an örtlich verdickten Führungsabschnitten (13b und 13d; 13e und 13f) des Schraubenschafts (13; 13') ausgebildeten zylindrischen Passflächen ausgeführt ist, welche durch einen mit einem geringeren Querschnitt als die Führungsabschnitte (13b und 13d; 13e und 13f) ausgeführten Dehnabschnitt (13c) des Schraubenschafts (13;13') voneinander getrennt sind und welche zum Zusammenwirken mit zwei entsprechenden, in Bohrungen (14,15) des Halsteils (3) und des Schaftteils (2) ausgebildeten Zentrierflächen bestimmt sind, wobei die eine Zentrierfläche in einem Bohrungsabschnitt (14d;15b) gebildet ist, der vollständig in einem der beiden Teile, Halsteil (3) oder Schaftteil (2), angeordnet ist und die andere Zentrierfläche in Bohrungsabschnitten (14b,15b) gebildet ist, die in beiden Teilen, Halsteil (3) und Schaftteil (2), angeordnet sind, und zwar in aneinander anschliessenden Bohrungsabschnitten (14b,15b) in der proximalen Endpartie (2a) des Schaftteils (2) und dem daran angrenzenden Abschnitt des Halsteils (3).

3. Bausatz nach einem der vorangehenden Ansprüche, **gekennzeichnet durch** mindestens einen an eine Seitenwand des Halsteils (3), lateral und/oder posterior und/oder anterior, ansetzbaren und am Halsteil (3) befestigbaren Füllkörper (17, 18) als Ersatz für eine fehlende Knochenpartie.

4. Bausatz nach Anspruch 3, **dadurch gekennzeichnet, dass** der Halsteil (3), oder der Füllkörper (17, 18), mit mindestens einer im wesentlichen in Richtung der Längsachse (L) des Schaftteils (2) verlaufenden, z.B. schwalbenschwanzförmig ausgebildeten, schienenartigen Führungspartie (16) ausgeführt ist und dass der Füllkörper (17, 18), bzw. der Halsteil (3), mit einer, entsprechenden nutenartigen Führungsbahn (21) ausgeführt ist.

5. Bausatz nach Anspruch 3 oder 4, **dadurch gekennzeichnet, dass** der Füllkörper (17, 18) mit einer im wesentlichen in Richtung der Längsachse (L) des Schaftteils (2) auf eine proximale Endpartie des Halsteils (3) aufsetzbaren Kragenpartie (20) ausgeführt ist.

6. Bausatz nach Anspruch 5, **dadurch gekennzeichnet, dass** der Halsteil (3) in seiner proximalen Endpartie mit einer Vertiefung (9) ausgeführt ist, die zur Aufnahme des Kopfs (12) der Dehnschaftschraube (11) und/oder zumindest eines Teils der Kragenpartie (20) des Füllkörpers (17, 18) bestimmt ist und dass ein diese Vertiefung (9) überdeckendes, gegen die Kragenpartie (20) verspannbares Abdeckelement (22) vorgesehen ist.

7. Femurkopfprothese aus einem Bausatz nach einem der vorangehenden Ansprüche.

## Claims

1. Kit of parts for a modular femur head prosthesis, in particular for a reoperation prosthesis, containing a distal shaft part (2) which can be anchored in a femur bone (1) and a neck part (3), which can be equipped with an artificial joint ball (10) and which can be fixed by means of a screw connection to the proximal end portion (2a) of the shaft part (2), wherein the screw connection for the connecting of the shaft part (2) and the neck part (3) comprises an extensible shaft screw (11) which can be elastically prestressed with a predetermined prestressing force and wherein the proximal end portion (2a) of the shaft part (2) and a connection portion (3a) of the neck part (3) which can be mounted onto this proximal end portion (2a) are executed with mutually supportable support surfaces (2c, 3c) set transverse to the longitudinal axis (L) of the shaft part (2), and also with guide surfaces (2d, 3d), which can be brought together in form-fitted manner and are orientated in the direction of the longitudinal axis (L), **characterized in that** the guide surfaces (2d, 3d) are formed on a spigot-like projection (2b) at the proximal end portion (2a) of the shaft part (2) and in a cutout (3b) in the connection portion (3a) of the neck part (3) formed to receive this projection (2a), and in that the proximal end portion (2a) of the shaft part (2) and its projection (2b) and also the connection portion (3a) of the neck part (3) mountable onto this end portion (2a) of the shaft part (2) and its cutout (3b) are each provided with an at least approximately rectangular cross-section, the larger longitudinal dimension (A) of which extends substantially in the lateral direction of the femur bone (1).

2. Kit of parts in accordance with claim 1, **characterized in that** the extensible shaft screw (11) is designed with two cylindrical locating surfaces formed at locally thickened guide sections (13b and 13d; 13e and 13f) of the screw shaft (13; 13'), which are separated from one another by an extensible section (13c) of the screw shaft (13; 13') executed with a smaller cross-section than the guide sections (13b and 13d; 13e and 13f) and which are intended to cooperate with two corresponding centering surfaces formed in bores (14, 15) of the neck part (3) and of the shaft part (2), with the one centering surface being formed in a bore section (14d, 15b) which is fully arranged in one of the two parts - neck part (3) or shaft part (2) and with the other centering surface being formed in bore sections (14b; 15b), which are arranged in the two parts neck part (3) and shaft part (2) and indeed in adjoining bore sections (14b, 15b) in the proximal end portion (2a) of the shaft part (2) and the adjoining section of the neck part (3).

3. Kit of parts in accordance with one of the preceding claims, **characterized by** at least one filling body (17, 18) as a replacement for a missing bone portion and mountable onto a sidewall of the neck part (3) - laterally and/or posterially and/or anterially - and securable to the neck part (3).

4. Kit of parts in accordance with claim 3, **characterized in that** the neck part (3) - or the filling bodies (17, 18) is executed with at least one rail-like guide portion (16) extending substantially in the direction of the longitudinal axis (L) of the shaft part (2) and, for example, formed in dovetail-like manner; and in that the filling bodies (17, 18) or the neck part (3) is executed with a corresponding groove-like guide track (21).

5. Kit of parts in accordance with claim 3 or 4, **characterized in that** the filling body (17, 18) is executed with a collar portion (20), which can be mounted substantially in the direction of the longitudinal axis (L) of the shaft portion (2) onto a proximal end portion of the neck part (3).

6. Kit of parts in accordance with claim 5, **characterized in that** the neck part (3) is executed in its proximal end portion with a recess (9), which is intended to receive the head (12) of the extensible shaft screw (11) and/or at least a part of the collar portion (20) of the filling body (17, 18) and in that a collar element (22) which covers over this recess (9) and which can be clamped against the collar portion (20) is provided.

7. Femur head prosthesis from a kit of parts in accordance with one of the preceding claims.

## Revendications

1. Eléments de construction pour une prothèse de tête fémorale modulaire, notamment pour une prothèse de réopération, comportant une partie de corps distale (2) pouvant être ancrée dans un os de fémur (1) et une partie de col (3) pouvant être fixée à la partie d'extrémité proximale (2a) de celle-ci par un assemblage par vissage, pouvant être munie d'une articulation sphérique artificielle (10), où l'assemblage par vissage pour relier la partie de corps (2) et la partie de col (3) comprend une vis à tige allégée (11) pouvant être précontrainte élastiquement selon une force de précontrainte prédéterminée, et où la partie d'extrémité proximale (2a) de la partie de corps (2) et une partie de raccordement (3a) de la partie de col (3) pouvant être placée sur cette partie d'extrémité proximale (2a) sont réalisées avec des faces d'appui (2c, 3c) placées transversalement à l'axe longitudinal (L) de la partie de corps (2), s'appuyant l'une sur l'autre et avec des faces de guidage (2d, 3d) orientées dans la direction de l'axe longitudinal (L), pouvant être réunies par concordance des formes, **caractérisés en ce que** les faces de guidage (2d, 3d) sont réalisées à un prolongement (2b) en forme de pivot à la partie d'extrémité proximale (2a) de la partie de corps (2) et dans un évidement (3b), réalisé pour la réception de ce prolongement (2a), dans la partie de raccordement (3a) de la partie de col (3), et en ce que la partie d'extrémité proximale (2a) de la partie de corps (2) et son prolongement (2b) ainsi que la partie de raccordement (3a) de la partie de col (3) pouvant être placée sur cette partie d'extrémité (2a) de la partie de corps (2) et son évidement (3b) sont réalisés chacun en une section transversale au moins approximativement rectangulaire, dont la dimension longitudinale plus grande (A) s'étend essentiellement dans la direction latérale de l'os de fémur (1).

2. Eléments de construction selon la revendication 1, **caractérisés en ce que** la vis à tige allégée (11) est réalisée avec deux surfaces d'ajustage cylindriques réalisées à des tronçons de guidage localement épaissis (13b et 13d ; 13e et 13f) de la tige de vis (13 ; 13'), qui sont séparées l'une de l'autre par un tronçon extensible (13c) de la tige de vis (13 ; 13') réalisé en une section transversale plus petite que les tronçons de guidage (13b et 13d ; 13e et 13f) et qui sont destinées à la coopération avec deux faces de centrage correspondantes, réalisées dans des perçages (14, 15) de la partie de col (3) et de la partie de corps (2), où une face de centrage est formée dans un tronçon de perçage (14d, 15b) qui est disposé complètement dans l'une des deux parties, partie de col (3) ou partie de corps (2), et l'autre face de centrage est formée dans des tronçons de perçage (14b, 15b) qui sont disposés dans les deux parties, partie de col (3) et partie de corps (2), à savoir dans des tronçons de perçage (14b, 15b) contigus dans la partie d'extrémité proximale (2a) de la partie de corps (2) et le tronçon avoisinant celle-ci de la partie de col (3).

3. Eléments de construction selon l'une des revendications précédentes, **caractérisés par** au moins un corps de remplissage (17, 18) applicable à une paroi latérale de la partie de col (3), côté latéral et/ou postérieur et/ou antérieur, et pouvant être fixé à la partie de col (3), comme remplacement d'une partie osseuse manquante.

4. Eléments de construction selon la revendication 3, **caractérisés en ce que** la partie de col (3) où le corps de remplissage (17, 18) est réalisé avec au moins une partie de guidage (16) en forme de rail, s'étendant sensiblement dans la direction de l'axe longitudinal (L) de la partie de corps (2), par exemple réalisée en forme de queue d'aronde, et en ce que le corps de remplissage (17, 18) respectivement la partie de col (3) est réalisée avec une voie de guidage correspondante (21) en forme de rainure.

5. Eléments de construction selon la revendication 3 ou 4, **caractérisés en ce que** le corps de remplissage (17, 18) est réalisé avec une partie de col (20) applicable sensiblement dans la direction de l'axe longitudinal (L) de la partie de corps (2) sur une partie d'extrémité proximale de la partie de col (3).

6. Eléments de construction selon la revendication 5, **caractérisés en ce que** la partie de col (3) est réalisée dans sa partie d'extrémité proximale avec un creux (9) qui est prévu pour la réception de la tête (12) de la vis à tige allégée (11) et/ou au moins d'une partie de la partie de col (20) du corps de remplissage (17, 18), et en ce qu'il est prévu un élément de recouvrement (22) recouvrant ce creux (9), pouvant être serré contre la partie de col (20).

7. Prothèse de tête fémorale réalisée par des éléments de construction selon l'une des revendications précédentes.
